## Europäisches Patentamt

## European Patent Office

## Office européen des brevets

(11) Veröffentlichungsnummer: **0 380 752**
**A1**

(19)

(12) # EUROPÄISCHE PATENTANMELDUNG

(21) Anmeldenummer: **89117927.7**

(51) Int. Cl.⁵: **G01N 33/28**

(22) Anmeldetag: **28.09.89**

(30) Priorität: **22.12.88 DE 3843243**

(43) Veröffentlichungstag der Anmeldung:
**08.08.90 Patentblatt 90/32**

(84) Benannte Vertragsstaaten:
**DE FR GB IT SE**

(71) Anmelder: **FEV Motorentechnik GmbH & Co. KG**
**Jülicher Strasse 342-352**
**D-5100 Aachen(DE)**

(72) Erfinder: **Pischinger, Franz, Prof.-Dr.**
**Im Erkfeld 4**
**D-5100 Aachen(DE)**
Erfinder: **Scheid, Ernst, Dr.-Ing.**
**Soerser Winkel 31**
**D-5100 Aachen(DE)**
Erfinder: **Hilger, Ulrich, Dr.-Ing.**
**Königstrasse 73B**
**D-5100 Aachen(DE)**
Erfinder: **Schmitz, Günter, Dr.-Ing.**
**Zehnthofweg 31**
**D-5100 Aachen(DE)**

(74) Vertreter: **Fischer, Friedrich B., Dr.-Ing.**
**Saarstrasse 71**
**D-5000 Köln 50 (Rodenkirchen)(DE)**

(54) **Vorrichtung zur Feststellung des Alkoholgehaltes und/oder des Heizwertes von Kraftstoffen.**

(57) Eine Vorrichtung zur Feststellung des Alkoholgehaltes und/oder des Heizwertes von Kraftstoffen durch Messung von Kenngrößen des Kraftstoffs in einer Meßzelle, in der ein von Kraftstoff mindestens teilweise benetzter Körper (Strömungskörper) angeordnet ist, wird insbesondere hinsichtlich der Meßgenauigkeit und der Schnelligkeit der Messung dadurch verbessert, daß der Strömungskörper (Innenkörper) auf seiner Außenseite eine erste Elektrode zur Dielektrizitätsmessung des Kraftstoffs und eine zweite Elektrode zur Messung der Leitfähigkeit des Kraftstoffs aufweist. Vorzugsweise bilden die erste Elektrode zur Messung der Kapazität, ihr Außenanschluß und die Wandung der Meßzelle ein mechanisch starres System. Auch kann es zweckmäßig sein, daß die Meßschaltung zur Messung zumindest der Kapazität unmittelbar am Gehäuse der Meßzelle angeordnet ist.

Fig. 1

Vorrichtung zur Feststellung des Alkoholgehaltes und/oder des Heizwertes von Kraftstoffen

Die Erfindung bezieht sich auf eine Vorrichtung zur Feststellung des Alkoholgehaltes und/oder des Heizwertes von Kraftstoffen durch Messung von Kenngrößen des Kraftstoffs in einer Meßzelle, in der ein von Kraftstoff mindestens teilweise benetzter Körper (Strömungskörper) angeordnet ist.

In Anbetracht der langfristig nur in begrenztem Umfang verfügbaren Reserven an fossiler Energie, insbesondere der aus Rohöl gewonnenen Kraftstoffe, und in Anbetracht der steigenden Anforderungen an den Umweltschutz, wird diesen Kraftstoffen in zunehmendem Maße Methyl- oder Äthylalkohol zugemischt. Dabei soll ein beliebiges Tanken sowohl von Reinkraftstoffen als auch von Mischkraftstoffen möglich sein. Bei höheren Alkoholanteilen ist dabei die Kenntnis des Mischungsverhältnisses erforderlich, um eine optimale Arbeitsweise der Brennkraftmaschine zu erreichen und dabei insbesondere eine genaue, den Betriebsverhältnissen angepaßte Kraftstoffzumessung zu ermöglichen. Besondere Probleme breitet dabei die laufende Feststellung des Alkoholgehaltes des der Brennkraftmaschine im Betrieb laufend zugeführten Kraftstoffs bei Fahrzeugmotoren, bei denen durch das beliebige Tanken der Kraftstoffarten jede mögliche Mischung erreicht werden kann.

In der deutschen Patentanmeldungt P 38 10 808.9 ist eine Vorrichtung der eingangs bezeichneten Art beschrieben, bei der in einer Meßzelle Kenngrößen des Kraftstoffs gemessen und in einer geeigneten Schaltung verarbeitet werden. Diese Schaltung ist ihrerseits mit einem Einspritzrechner verbunden, dessen Ergebnisse zur Steuerung einer Kraftstoffzumeßeinrichtung verwertet werden.

Es hat sich gezeigt, daß bei Meßeinrichtungen dieser Art eine hohe Genauigkeit der Messung, aber auch eine schnelle Bereitstellung der Meßwerte erforderlich ist, um die Kraftstoffzumessung schnell wechselnden Betriebsbedingungen anpassen zu können.

Der Erfindung liegt die Aufgabe zugrunde, eine Vorrichtung zur Feststellung des Alkoholgehaltes und/oder des Heizwertes von Kraftstoffen gemäß dem Oberbegriff des Anspruchs 1 zu schaffen, welche eine schnelle Bereitstellung der Meßwerte bei hoher Genauigkeit der Messung ermöglicht.

Gemäß der Erfindung ist vorgesehen, daß bei einer Vorrichtung zur Feststellung des Alkoholgehaltes und/oder des Heizwertes von Kraftstoffen der eingangs beschriebenen Art der Strömungskörper (Innenkörper) auf seiner Außenseite eine erste Elektrode zur Dielektrizitätsmessung des Kraftstoffs und eine zweite Elektrode zur Messung der Leitfähigkeit des Kraftstoffs aufweist. Vorzugsweise bilden dabei die erste Elektrode zur Messung der Kapazität, ihr Außenanschluß und die Wandung der Meßzelle ein mechanisch starres System. Dabei kann es sowohl hinsichtlich der erreichbaren Genauigkeit der Messung und ihrer Auswertung als auch hinsichtlich der Wirtschaftlichkeit der Fertigung und der Raumausnutzung vorteilhaft sein, daß die Meßschaltung zumindest der Kapazität unmittelbar am Gehäuse der Meßzelle angeordnet ist.

Gemäß einer bevorzugten Ausführungsform ist vorgesehen, daß die für eine definierte Kapazität wichtige Zentrierung des als Innenelektrode dienenden Innenkörpers über einen Käfig aus nichtleitendem Material erfolgt, der gleichzeitig auch als Halterung für den Innenkörper dienen kann.

Auch kann die Meßzelle zur Verbesserung des Meßvorganges und der rechnerischen Auswertung einen zusätzlichen Sensor zur Messung der Temperatur des durch die Meßzelle strömenden Kraftstoffs aufweisen.

Der Strömungskörper (Innenkörper) kann aus einem glasfaserverstärkten PTFE-Kunststoff (Polytetrafluoräthylen) bestehen, oder auch aus keramischem Material, auf dessen Außenseite die Elektroden angebracht sind. Auch kann er aus Stahl bestehen, auf dem die Elektroden isoliert angeordnet sind.

Für die Abmessungen und die Anordnung der Elektroden hat es sich als zweckmäßig erwiesen, daß die Fläche der zur Dielektrizitätsmessung bestimmten Elektrode mindestens das zweifache der zur Leitfähigkeitsmessung verwendeten Elektrode beträgt, und daß die zur Leitfähigkeitsmessung bestimmte Elektrode in Strömungsrichtung des Kraftstoffs im Abstand von der zur Dielektrizitätsmessung bestimmten Elektrode angeordnet ist.

Ausführungsbeispiele der Erfindung werden nachfolgend anhand der Zeichnung näher beschrieben.

Fig. 1 zeigt vereinfacht und im wesentlichen schematisch einen Längsschnitt durch eine erste Ausführungsform einer Meßzelle.

Fig. 2 zeigt in der gleichen Art der Darstellung eine weitere Ausführungsform.

Fig. 3 zeigt einen Querschnitt durch die in Fig. 2 dargestellte Ausführungsform.

Fig. 4 zeigt eine Einzelheit der Ausführungsform gemäß Fig. 2.

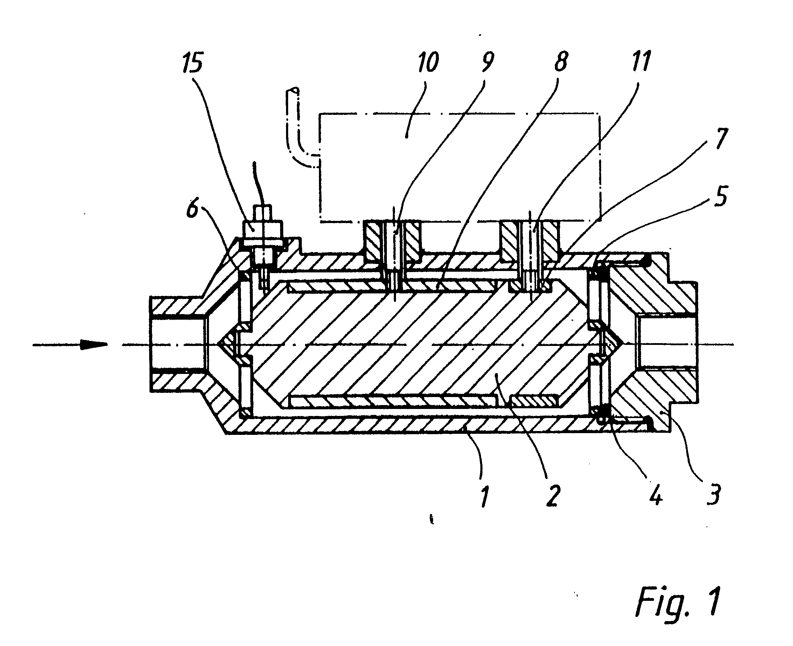

Fig. 1 zeigt im Längsschnitt eine Meßzelle, die zur Feststellung des Alkoholgehaltes und/oder des Heizwertes von Kraftstoffen geeignet ist, und zwar insbesondere mittels einer kondensatorischen Dielektrizitätsmessung. Die Meßzelle enthält ein elektrisch leitfähiges Gehäuse 1, in dem sich ein im wesentlichen zylindrischer Strömungskörper 2 in zentrischer Anordnung befindet. Eine Fixierung des Strömungskörpers 2 wird durch einen Verschlußstopfen 3 erreicht, der über eine elastische Anlagescheibe 4, z.B. über einen PTFE-Ring, auf ein Halterungsteil 5 drückt und dadurch den Strömungskörper 2 gegen einen im Gehäuse 1 gelagerten Halter 6 preßt. Der Verschlußstopfen 3 dient gleichzeitig zum Anschluß der Kraftstoffleitung.

Auf der Außenseite des Strömungskörpers 2, der gegebenenfalls auch geteilt ausgebildet sein kann, befinden sich zur Dielektrizitäts- bzw. zur Leitfähigkeitsmessung verwendete Elektroden 8 und 7 aus elektrisch leitfähigem Material. Die Größe der Oberfläche der zur kapazitiven Dielektrizitätsmessung verwendeten Plattenflächen hängt von der minimal für die elektronische Meßschaltung erforderlichen Kapazität ab, während für die Leitfähigkeitsmessung eine relativ kleine Elektrodenoberfläche ausreicht. Für die Messung der Dielektrizitätskonstanten des zwischen der durch das Gehäuse 1 der Meßzelle gebildeten äußeren Elektrode und der auf dem Strömungskörper befindlichen Gegenelektrode 8 strömenden Kraftstoffs ist es von besonderem Vorteil, wenn die elektrische Verbindung 9 der inneren Elektrode mit der elektronischen Meßschaltung eine unter allen Betriebsbedingungen konstante Kapazität aufweist. Dies wird dadurch erreicht, daß die elektrische Verbindung der Elektrode 8 mit Meßschaltung 10 starr ausgeführt wird. Hierzu wird z.B. ein PTFE-ummantelter Gewindestift durch die Gehäusedurchführung mit der Elektrode 8 verschraubt, wobei zur Fixierung und Abdichtung der elektrischen Verbindung im Gehäuse 1 eine Schneidring-Rohrverschraubung verwendet werden kann. Der elektrische Anschluß 11 der Elektrode 7 zur Leitfähigkeitsmessung kann anstelle der dargestellten starren Verbindung auch mittels eines flexiblen Leiters erfolgen. Die Meßschaltung zur Ermittlung der Kapazität wird in vorteilhafter Weise insbesondere zur Vermeidung von Störeinflüssen unmittelbar am Gehäuse 1 der Meßzelle angeordnet. Eine Möglichkeit hierzu besteht darin, die Meßschaltung 10 gemäß Fig. 1 unmittelbar auf den Meßelektrodenanschlüssen 9 und 11 zu befestigen. Für das Gehäuse 1, wie auch für den Strömungskörper 2 werden alkoholresistente Materialien, wie z.B. hochlegierte, korrosionsbeständige Stähle verwendet. Auch kann der Strömungskörper 2 aus PTFE-Kunststoff oder keramischem Material hergestellt sein. In diesem Falle sind die Innenelektroden 7 und 8 auf der Außenseite des Strömungskörpers 2, z.B. in Form von Stahlringen, angebracht. Auch kann der Strömungskörper 2 aus Stahl bestehen; die Elektroden 7 und 8 sind dann mit einer entsprechenden Isolierung auf dem Strömungskörper 2 anzuordnen.

Wie Fig. 1 zeigt, kann es zweckmäßig sein, zusätzlich einen Sensor 15 zur Messung der Temperatur des durch die Meßzelle strömenden Kraftstoffs vorzusehen, um den Temperatureinfluß bei der Auswertung eliminieren zu können.

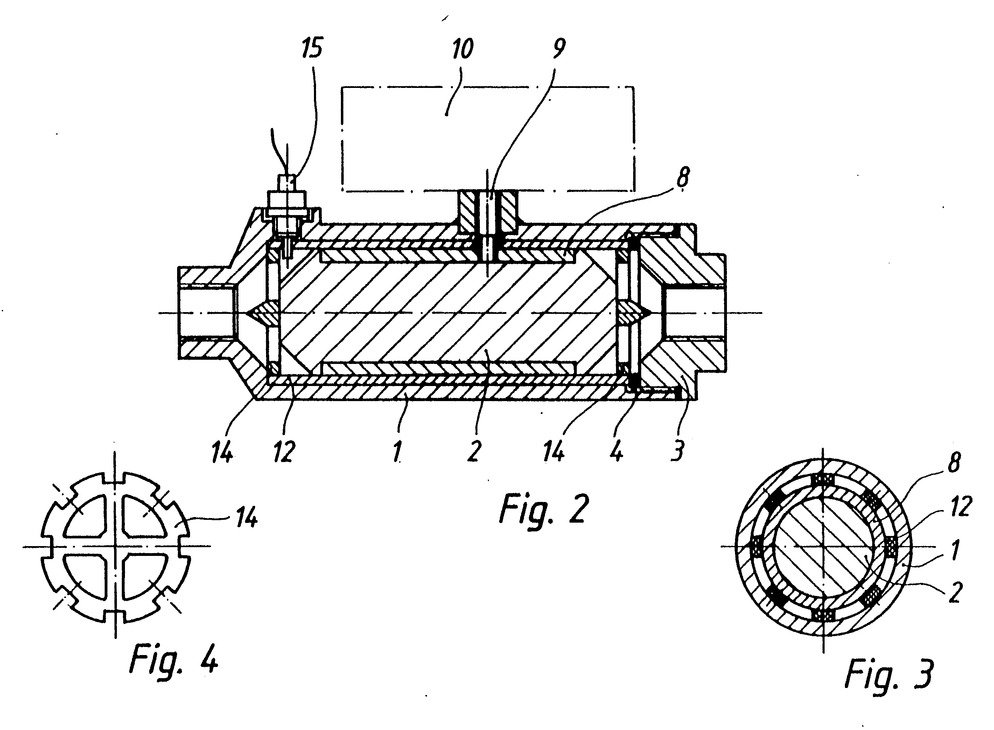

Eine weitere bevorzugte Ausführungsform ist in den Figuren 2-4 dargestellt. Der Strömungskörper 2 ist von einem Käfig 12 aus elektrisch nichtleitendem Material umgeben, der bündig an der Innenwand des Gehäuses 1 anliegt. Hierdurch wird in vorteilhafter Weise erreicht, daß zwischen der Innenelektrode 8 des Strömungskörpers 2 und der durch das Gehäuse 1 gebildeten Außenelektrode über die gesamte Länge ein konstanter, genau definierter Abstand eingehalten wird, so daß die für die Kapazitätsmessung besonders kritische Serienstreuung gering gehalten ist. Der Käfig 12 besteht aus mindestens drei Gitterelementen, die am Ende von einer Scheibe 14 (Fig. 4) gehalten werden, die gleichzeitig auch den Strömungskörper (Innenkörper) 2 hält. Um besonders kleine Fertigungsstreuungen zu erhalten, kann es dabei zweckmäßig sein, daß die Gitterelemente aus mit hoher Genauigkeit vorgefertigten Kunststoffelementen hergestellt werden.

## Ansprüche

1. Vorrichtung zur Feststellung des Alkoholgehaltes und/oder des Heizwertes von Kraftstoffen durch Messung von Kenngrößen des Kraftstoffs in einer Meßzelle, in der ein von Kraftstoff mindestens teilweise benetzter Körper (Strömungskörper) angeordnet ist, dadurch gekennzeichnet, daß der Strömungskörper (Innenkörper) auf seiner Außenseite eine erste Elektrode zur Dielektrizitätsmessung des Kraftstoffs und eine zweite Elektrode zur Messung der Leitfähigkeit des Kraftstoffs aufweist.

2. Vorrichtung nach Anspruch 1, dadurch gekennzeichnet, daß die erste Elektrode zur Messung der Kapazität, ihr Außenanschluß und die Wandung der Meßzelle ein mechanisch starres System bilden.

3. Vorrichtung nach Anspruch 1 oder 2, dadurch gekennzeichnet, daß die Meßschaltung zur Messung zumindest der Kapazität unmittelbar am Gehäuse der Meßzelle angeordnet ist.

4. Vorrichtung nach einem der Ansprüche 1-3, gekennzeichnet durch einen Käfig aus nichtleitendem Material zur Sicherung der für die Kapazitätsmessung erforderlichen Zentrierung des Strömungskörpers (Innenkörpers).

5. Vorrichtung nach Anspruch 4, dadurch gekennzeichnet, daß der Käfig aus nichtleitendem Material zugleich als Halterung für den Strömungskörper (Innenkörper) dient.

6. Vorrichtung nach einem der Ansprüche 1-5, dadurch gekennzeichnet, daß die Meßzelle einen zusätzlichen Sensor zur Messung der Temperatur des durch die Meßzelle strömenden Kraftstoffs aufweist.

7. Vorrichtung nach einem der Ansprüche 1-6, dadurch gekennzeichnet, daß der Strömungskörper (Innenkörper) aus einem glasfaserverstärkten PTFE-Kunststoff (Polytetrafluoräthylen) besteht.

8. Vorrichtung nach einem der Ansprüche 1-6, dadurch gekennzeichnet, daß der Strömungskörper (Innenkörper) aus keramischem Material besteht, auf dessen Außenseite die Elektroden angebracht sind.

9. Vorrichtung nach einem der Ansprüche 1-6, dadurch gekennzeichnet, daß der Strömungskörper (Innenkörper) aus Stahl besteht, auf dem die Elektroden isoliert angeordnet sind.

10. Vorrichtung nach einem der Ansprüche 1-9, dadurch gekennzeichnet, daß die Fläche der zur Dielektrizitätsmessung bestimmten Elektrode mindestens das zweifache der zur Leitfähigkeitsmessung bestimmten Elektrode beträgt.

11. Vorrichtung nach einem der Ansprüche 1-10, dadurch gekennzeichnet, daß die zur Leitfähigkeitsmessung bestimmte Elektrode in Strömungsrichtung des Kraftstoffs im Abstand von der zur Dielektrizitätsmessung bestimmten Elektrode angeordnet ist.

Fig. 1

EP 0 380 752 A1

Fig. 2

Fig. 4

Fig. 3

EP 0 380 752 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | PATENT ABSTRACTS OF JAPAN, Band 5, Nr. 175 (P-88)[847], 11. November 1981; & JP-A-56 104 243 (HITACHI SEISAKUSHO K.K.) 19-08-1981 --- | 1,3,6,8 | G 01 N 33/28 |
| A | IDEM --- | 2 | |
| Y | FR-A-2 409 389 (TNO) * Seite 3, Zeile 20 - Seite 4, Zeile 6; Seite 5, Zeile 28 - Seite 6, Zeile 13; Figur 2 * | 1,3,6,8 | |
| A | --- | 2 | |
| Y | IEEE TRANS. VEHICULAR TECHN., Band VT-27, Nr. 3, August 1978, Seiten 142-144, IEEE, New York, US; J.W. HILE et al.: "An on-board sensor for percent alcohol" * Das ganze Dokument. * --- | 6 | |
| A | IDEM --- | 1 | RECHERCHIERTE SACHGEBIETE (Int. Cl.5) |
| Y | US-A-2 892 976 (THAYER) * Spalte 2, Zeilen 47-71; Spalte 4, Zeilen 34-45 * | 8 | G 01 N |
| A | --- | 1,2,10 | |
| Y | PATENT ABSTRACTS OF JAPAN, Band 6, Nr. 133 (P-129)[1011], 20. Juli 1982; & JP-A-57 57 253 (NISSAN JIDOSHA K.K.) 06-04-1982 --- | 3 | |
| A | IDEM ----- | 1,6 | |

Der vorliegende Recherchenbericht wurde für alle Patentansprüche erstellt

| Recherchenort | Abschlußdatum der Recherche | Prüfer |
|---|---|---|
| DEN HAAG | 26-04-1990 | BOSMA R.A.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes Dokument